# EUROPÄISCHE PATENTSCHRIFT

(11) **EP 0 676 175 B1**
(45) Veröffentlichungstag und Bekanntmachung des Hinweises auf die Patenterteilung: **06.05.1998**
(21) Anmeldenummer: 95102232.6
(22) Anmeldetag: 17.02.1995
(51) Int. Cl.: A61B 17/22

(54) **Einrichtung zum Entfernen von Körpersteinen**
Device for removing calculi
Dispositif pour évacuer des concrétions calcaires corporelles

(30) Priorität: 22.02.1994 DE 4405656
(43) Veröffentlichungstag der Anmeldung: 11.10.1995
(73) Patentinhaber: Ferton Holding, CH-2800 Delemont (CH)
(72) Erfinder: Schulz, Manfred, D-88662 Überlingen (DE); Merkle, Wolfgang, D-52441 Linnich (DE)
(74) Vertreter: Gauger, Hans-Peter, Dipl.-Ing.

(56) Entgegenhaltungen:
- WO-A-93/08750
- DE-A- 3 720 424
- GB-A- 2 268 883
- US-A- 5 069 664

## Beschreibung

Die Erfindung bezieht sich auf eine Einrichtung zum Entfernen von Körpersteinen nach dem Oberbegriff des Anspruchs 1.

Aus der EP 0 317 507 B1 ist ein Lithotripter bekannt, bei dem eine Stoßwellen übertragende Sonde durch ein pneumatisch hin und her bewegtes Projektil mit Stoßenergie beaufschlagt wird, die bei einer Berührung der Sondenspitze mit einem Körperstein zu einer Zertrümmerung desselben führt. Die mit einem solchen Lithotripter intrakorporal durchgeführte Steinzertrümmerung wird dabei unter Verwendung eines Endoskops verwirklicht, in dessen Arbeitskanal die Sonde des Lithotripters so weit eingeführt wird, bis die Sondenspitze etwas über das Ende des Arbeitskanals vorsteht und damit eine unter einer Sichtkontrolle ständig beizubehaltende Berührung der Sondenspitze mit dem zu zertrümmernden Stein möglich wird.

Das Arbeiten mit solchen intrakorporalen Lithotriptern hat sich bis heute weitgehend bewährt, jedoch ist damit noch der Nachteil verbunden, daß es für eine optimale Steinzertrümmerung erforderlich ist, die Relativlage der Sondenspitze zu dem zu zertrümmernden Stein ständig zu korrigieren, damit auch bei abnehmender Größe der Steinfragmente deren am wenigsten widerstandsfähige Angriffspunkte für eine entsprechend optimale Steinzertrümmerung bis hin zu einer Steingröße aufgefunden werden können, die von dem Arzt als spontan abgangsfähig beurteilt werden kann, insbesondere für einen Abgang bei gleichzeitiger Einlage einer Harnleiterschiene. Die eigentliche Steinzertrümmerung erfährt damit notwendigerweise eine Verknüpfung mit dieser Beurteilung einer Entfernungsmöglichkeit der zertrümmerten Steine mittels einer eingelegten Harnleiterschiene.

Aus der DE 35 45 176 C2 ist ein Endoskop bekannt, in dessen Arbeitskanal wahlweise eine mit einer Spreizeinrichtung verbundene Endoskopoptik oder eine mit einer Ultraschall-Sonotrode verbundene Arbeitsoptik einsetzbar ist. Auf das freie Ende des Arbeitskanals ist eine aus einem gummielastisch dehnbaren Material bestehende Aufnahmetasche aufgesetzt. Diese Aufnahmetasche weist eine mittels der Spreizeinrichtung entgegen der elastischen Rückstellkraft des Taschenmaterials aufweitbare Aufnahmeöffnung auf, über die ein zu zertrümmernder Stein für seine Zertrümmerung innerhalb der Aufnahmetasche aufgenommen werden kann. Die Steinzertrümmerung wird mit einer Ultraschall-Sonotrode vorgenommen, die anschließend anstelle der Spreizeinrichtung in den Arbeitskanal des Endoskops eingeführt wird, wobei zu diesem Zeitpunkt die Aufnahmeöffnung an der Aufnahmetasche wieder geschlossen ist und so ein Austreten der bei der Zertrümmerung erhaltenen Steinfragmente verhindert wird. Nach dem Entfernen der Sonotrode können dann die Steinfragmente mittels der Aufnahmetasche aus dem Arbeitskanal des Endoskops entfernt werden.

Aus der DE 37 20 424 C2 ist eine Ultraschall-Sonotrode bekannt, bei welcher ein zur Übertragung der damit erzeugten Ultraschallwellen vorgesehenes flexibles Schwingungsübertragungsteil von einem rohrförmigen Schutzmantel umgeben ist, über welchen unter Vermittlung der Sonotrode eine Perfusionsflüssigkeit in die Körperhöhle geliefert wird, wo sich ein mittels der Sonotrode zu zertrümmender Stein befindet. An der Außenseite des Schutzmantels ist ein Ringkanal im Zusammenwirken entweder mit einem weiteren Kunststoffschlauch oder mit der umgebenden Wand des Arbeitskanals eines Endoskops ausgebildet, in welchen das mit dem Schutzmantel geschützte Schwingungsübertragungsglied eingeführt wird, wobei dieser Ringkanal für ein Absaugen der Perfusionsflüssigkeit und der Steinfragmente dient, die bei dem Arbeiten mit der Sonotrode anfallen. Für den Schutzmantel ist dabei gleichzeitig eine solche Anordnung bezüglich eines als Handstück benutzbaren Gehäuses der Sonotrode gewählt, daß es durch ein Hin- und Herschieben dieses Schutzmantels relativ zu dem Schwingungsübertragungsteil möglich ist, dessen Endabschnitt für die Zertrümmerung eines Steines freizulegen.

Bei einer nach dem Oberbegriff des Anspruchs 1 bekannten Einrichtung gemäß der GB-A-2 268 883 ist für eine Entfernungsmöglichkeit der Steinfragmente, die mit der Übertragung der Stoßwellen durch die in den Arbeitskanal eines Endoskops eingesetzte Sonde eines Lithotripters bspw. der durch die EP 0 317 507 B1 bekannten Art erhalten wurden, ein Absperrorgan in der Ausbildung eines Y-förmigen Anschlußstückes vorgesehen. Dieses Anschlußstück ist mit seinem einen Schenkel der Y-Form in einer solchen umgekehrten Anordnung an die erste Anschlußbohrung des Saugkanals angeschlossen, daß sich für seinen zweiten Schenkel eine zur Atmosphäre hin abgewinkelte Öffnung ergibt. Bei einer Verbindung des Schlauchanschlusses der Saugpumpe mit der Basis dieses Y-förmigen Anschlußstückes muß diese Offnung von der Bedienungsperson mit einer Hand verschlossen werden, damit sich der Saugdruck der ständig eingeschalteten Saugpumpe hin zu dem Saugkanal für ein Ansaugen der Steinfragmente entwickeln kann. Bei dieser bekannten Einrichtung wird im übrigen die Sonde des Lithotripters über eine an dem Ende des Führungsrohres angeordnete Dichtungsmanschette direkt in das Führungsrohr abgedichtet eingeführt. Die mit dem vorerwähnten Absperrorgan versehene erste Anschlußbohrung an den Saugkanal des Führungsrohres ist dabei in der unmittelbaren Nähe von diesem Ende des Führungsrohres vorgesehen, das rückwärts aus dem Arbeitskanal des Endoskops für die Verbindung mit einem Lithotripter vorsteht. Eine axiale Verstellbarkeit der Sonde des Lithotripters relativ zu dem Führungsrohr ist dabei nicht realisiert.

Der vorliegenden Erfindung liegt die Aufgabe zugrunde, eine Einrichtung zum Entfernen von Körpersteinen der durch den Oberbegrifff des Anspruches 1 angegeben Art derart auszubilden, daß sich eine weniger problematische Steinzertrümmerung ergibt und sich gleichzeitig die Entfernung der dabei erhaltenen Steinfragmente als weniger kritisch erweist. Diese Aufgabe wird erfindungsgemäß gelöst mit einer Einrichtung der durch den Anspruch 1 angegebenen Art.

Durch die erfindungsgemäß vorgesehene Eingliederung der Kupplungseinrichtung und deren axial verstellbare Ausbildung für die Möglichkeit einer Veränderung der Relativlage der Sonde des Lithotripters relativ zu dem Führungsrohr wird für die mit der Sonde übertragenen Stoßwellen eine verbesserte Steinzertrümmerung erhalten. Andererseits wird damit nicht die Möglichkeit benachteiligt, jeden für eine Zertrümmerung durch die Sondenspitze anvisierten Körperstein durch den an dem Saugkanal anliegenden und mit dem regelbaren Absperrorgan in der bevorzugten Ausbildung eines Trompetenventils auch regelbaren Saugdruck an dem Ende des Führungsrohres festhalten zu können. Die bei der Steinzertrümmerung anfallenden Steinfragmente können dann entweder unmittelbar über den Saugkanal abgesaugt werden, wobei dafür primär der Ringkanal zur Verfügung steht, der zwischen dem Führungsrohr und der in dieses koaxial eingeführten Sonde des Lithotripters ausgebildet ist. Alternativ steht aber für die Entfernung der Steinfragmente auch noch der gesamte Hohlraum des Führungsrohres zur Verfügung, sobald der Lithotripter an der Kupplungseinrichtung von dem Führungsrohr gelöst und dann die Sonde aus dem Führungsrohr zurückgezogen worden ist.

Ein Ausführungsbeispiel der erfindungsgemäßen Einrichtung zum Entfernen von Körpersteinen ist in der Zeichnung schematisch dargestellt und wird nachfolgend näher beschrieben.
Es zeigt
- Fig. 1: einen Axialschnitt der Einrichtung mit einer gleichzeitigen Darstellung ihrer Befestigung an dem Handstück eines Lithotripters gemäß einer ersten Ausführungsform,
- Fig. 2: einen Längsschnitt der Kupplungseinrichtung, mittels welcher ein Anschlußstück des Führungsrohres der Einrichtung gemäß Fig. 1 an dem Handstück des Lithotripters befestigt werden kann, und
- Fig. 3: einen Längsschnitt der Kupplungseinrichtung gemäß einer alternativen Ausbildung.

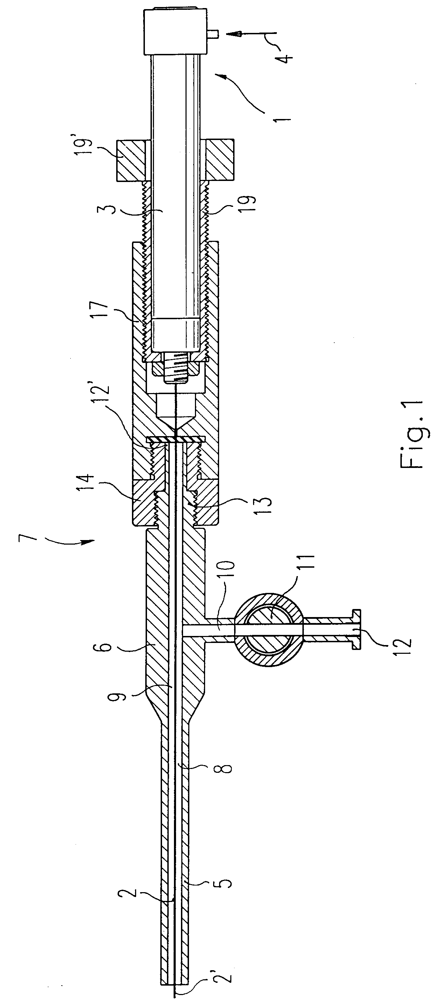

In Fig. 1 ist ein intrakorporaler Lithotripter 1 mit einer Stoßwellen übertragenden Sonde 2 schematisch gezeigt, welcher eine Ausbildung gemäß der EP 0 317 507 B1 aufweist und von der Firma EMS Electro Medical Systems S.A., Le Sentier (Schweiz), unter dem Markennamen "Swiss LITHOCLAST" als ein sog. "ballistischer" Lithotripter hergestellt und vertrieben wird. Der Lithotripter 1 weist ein zylindrisch ausgebildetes Handstück 3 auf und ist mit einem mit einer Druckluftquelle zu verbindenden Druckluftanschluß 4 versehen, sodaß damit ein Antrieb für ein hin und her bewegtes Projektil zur Verfügung steht, mit welchem auf die Sonde 2 eine Stoßenergie übertragen wird.

Die Sonde 2 ist in einem koaxial angeordneten Führungsrohr 5 aufgenommen, über welches die Sondenspitze 2' für eine Berührungsmöglichkeit mit einem zu zertrümmernden Stein nach vorne vorsteht, wenn dafür das Führungsrohr 5 zusammen mit dem an einem Anschlußstück 6 über eine Kupplungseinrichtung 7 befestigten Lithotripter 1 in den Arbeitskanal eines Endoskops (nicht dargestellt) eingeführt ist. Das Führungsrohr 5 ist so bemessen, daß zwischen ihm und der Sonde 2 ein Ringkanal 8 ausgebildet ist, der eine Verlängerung in einer Durchgangsbohrung 9 des Anschlußstückes 6 aufweist und über eine dazu quer ausgerichtete Anschlußbohrung 10 an eine Saugpumpe (nicht dargestellt) anschließbar ist. Der Ringkanal 8 bildet einen Ansaugkanal, welcher bei angeschlossener Saugpumpe zum Beginn der Steinzertrümmerung den Stein an dem Ende des Führungsrohres 5 festhält, sodaß die an der Sondenspitze 2' zur Übertragung kommende Stoßenergie eine genau gezielte Einwirkung auf den Stein erfahren kann. Daneben bildet dieser Ringkanal 8 einen Absaugkanal für die Steinfragmente einer Größe kleiner als der Querschnitt dieses Ringkanals 8. Als ein Beispiel kann die Absaugmöglichkeit von Steinfragmenten mit einem Durchmesser bis max. 2 mm bzw. bis max. 1.5 mm beim Arbeiten mit einer Sonde mit einem Durchmesser von entweder 1.6 mm oder 2 mm angegeben werden, wenn die Sonde 2 in ein Führungsrohr 5 der Größe 12 Fr., einem in der Endoskopie gebräuchlichen Umfangsmaß, eingeführt ist.

An der mit der Durchgangsbohrung 9 des Anschlußstückes 6 verbundenen Anschlußbohrung 10, die auch geneigt zu der mit dem Saugkanal des Führungsrohres 5 axial fluchtenden Durchgangsbohrung ausgerichtet sein kann, ist ein Absperrorgan 11 in der Ausbildung eines sog. Trompentenventils angeordnet, mittels welchem bei angeschlossener Saugpumpe der an dem Saugkanal anliegende Saugdruck regelbar bzw. absperrbar ist. In Fig. 1 ist deshalb ein axial verschiebbarer Ventilkörper für das Absperrorgan 11 gezeigt, welcher eine auf die Anschlußbohrung 10 ausrichtbare Bohrung aufweist. Mit einer axialen Verschiebung des Ventilkörpers wird die Fluidverbindung zu einem ersten Luer-Lock-Anschluß 12 gesteuert, der für einen Schlauchanschluß der Saugpumpe vorgesehen ist.

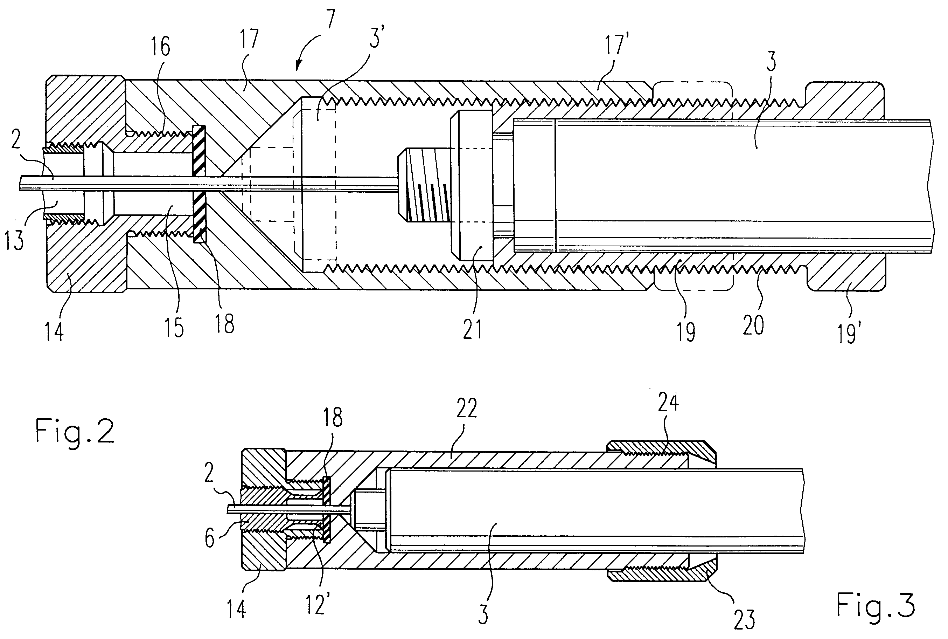

Das Anschlußstück 6 ist weiterhin mit einem Gewindeansatz 13 ausgebildet, mit welchem ein koaxiales Verbindungsstück 14 der Kupplungseinrichtung 7 verschraubt ist. Wie aus der vergrößerten Darstellung der Fig. 2 näher ersichtlich ist, weist auch dieses Verbindungsstück 14 eine Durchgangsbohrung 15 auf, die in der axialen Verlängerung der Durchgangsbohrung 9 des Anschlußstückes 6 eine zweite Anschlußbohrung an den Saugkanal des Führungsrohres 5 bildet, und einen zweiten Luer-Lock-Anschluß 12' umgibt, der an dem Gewindeansatz 13 ausgebildet ist. Bei entferntem Lithotripter 1 kann der Schlauchanschluß der Saugpumpe alternativ an den Luer-Lock-Anschluß 12' angeschlossen werden, womit Steinfragmente bis hin zu einer Größe von max. 3.5 mm über den dabei als Absaugkanal zur Verfügung stehenden axialen Hohlraum des Führungsrohres 5 abgesaugt werden können.

Das Verbindungsstück 14 der Kupplungseinrichtung 7 ist im übrigen an einem eine Teillänge der Durchgangsbohrung 15 umgebenden Ansatz mit einem Außengewinde 16 versehen, auf welches das mit einem komplementären Innengewinde versehene Anschlußende einer Kupplungshülse 17 aufschraubbar ist. In das Anschlußende dieser Kupplungshülse 17 ist ein Silikon-Dichtungsring 18 eingesetzt, der durch die Verschraubung mit dem Außengewinde 16 des Verbindungsstückes 14 fixiert ist und den mit dem Führungsrohr 5 ausgebildeten Saugkanal gegen die Sonde 2 abdichtet, die durch das Mittenloch dieses Dichtungsrings 18 hindurchgeführt ist.

Die Kupplungshülse 17 ist mit dem Handstück 3 des Lithotripters kraftschlüssig verbunden, wobei die Kraftschluß-Verbindung für eine axiale Verstellbarkeit der Sonde 2 relativ zu dem Führungsrohr 5 ausgebildet ist, damit das Maß, um welches die Sondenspitze 2' über das Ende des Führungsrohrs 5 vorsteht, verändert werden kann. Bei der in Fig. 2 gezeigten Ausführungsform ist die Kraftschluß-Verbindung der Kupplungshülse 17 mittels einer Gewindehülse 19 verwirklicht, die an einem Stellgewinde 20 mit dem Befestigungsende 17' der Kupplungshülse 17 relativ verstellbar verschraubt ist. Die Gewindehülse 19 ist auf das Handstück 3 des Lithotripters koaxial aufgesetzt und mittels einer auf einen Gewindeansatz des Handstückes aufgeschraubten Mutter 21 gegen eine axiales Verschieben gesichert. Wenn daher die Kupplungshülse 17 und die Gewindehülse 19 relativ zueinander gedreht werden, wobei die Drehung der Gewindehülse 19 an einem Bund 19' zu bewirken ist, dann kann sich das Handstück 3 des Lithotripters in der Kupplungshülse 17 bis maximal in die gestrichelt gezeichnete Position 3' axial vorschieben, in welcher dann die Sondenspitze 2' am weitesten über das Ende des Führungsrohres 5 axial vorsteht. Weil die Einhaltung eines bestimmten Maßes für das Vorstehen der Sondenspitze 2' über das Ende des Führungsrohres 5 im Einzelfall von Bedeutung sein kann, ist das Stellgewinde 20 zweckmäßig für ein bestimmtes axiales Einstellmaß der Sondenspitze relativ zu dem Ende des Führungsrohres geeicht, wobei an dem Handstück 3 eine Meßskala (nicht dargestellt) angebracht ist, an welcher das Verstellmaß abgelesen werden kann. Die Meßskala ist dabei auf eine Nullposition der Kupplungshülse 17 oder eine Nullposition der Sondenspitze 2' relativ zu dem Führungsrohr 5 bezogen und für das axiale Einstellmaß der Sondenspitze geeicht.

Bei der in Fig. 3 gezeigten alternativen Ausführungsform ist eine Kupplungshülse 22 verwendet, die ebenfalls an einem Verbindungsstück 14 unter Zwischenfügung eines Dichtungsringes 18 verschraubt ist. Die Kupplungshülse 22 ist hier abweichend direkt auf das Handstück 3 des Lithotripters axial aufgesetzt und mittels einer Überwurf-Spannmutter 23, die auf ein Außengewinde 24 am Befestigungsende der Spannhülse aufgeschraubt wird, gegen das Handstück festspannbar. Bei gelöster Spannmutter 23 kann somit hier die Kupplungshülse 22 relativ zu dem Handstück 3 und damit relativ zu der Sonde 2 verstellt werden, um auch damit wieder das axiale Einstellmaß für die Sondenspitze 2' relativ zu dem Ende des Führungsrohres 5 zu verändern. Die axiale Verstellung der Kupplungshülse 22 relativ zu dem Handstück 3 wird dabei ebenfalls entlang einer Meßskala (nicht dargestellt) vorgenommen, die an dem Handstück 3 angebracht und für das Einstellmaß der Sondenspitze 2' in Bezug auf eine Nullposition wie vorerwähnt geeicht ist.

## Patentansprüche

1. Einrichtung zum Entfernen von Körpersteinen, wie Nieren-, Harnleiter- oder Blasensteinen, unter Verwendung eines intrakorporalen Lithotripters (1) mit einer Stoßwellen übertragenden Sonde (2), die für eine Steinzertrümmerung in den Arbeitskanal eines Endoskops eingesetzt wird, bestehend aus
- einem Führungsrohr (5) mit einem Außendurchmesser und mit einem Innendurchmesser, wobei der Außendurchmesser für ein koaxiales Einführen des Führungsrohres (5) in den Arbeitskanal des Endoskops und der Innendurchmesser für eine koaxiale Aufnahme der Sonde (2) des Lithotripters (1) angepaßt sind;
- einem Saugkanal (8, 9, 10, 15), der als ein Ringkanal (8) zwischen dem Führungsrohr (5) und der in das Führungsrohr eingesetzten Sonde (2) des Lithotripters (1) ausgebildet ist, wobei mit einer Teillänge (9) des Saugkanals eine quer oder geneigt zu der Achse dieser Teillänge ausgerichtete erste Anschlußbohrung (10) verbunden ist, an welche ein Schlauchanschluß einer Saugpumpe anschließbar ist; und
- einem Absperrorgan (11), das an der ersten Anschlußbohrung (10) angeordnet ist und bei angeschlossener Saugpumpe einen von der Saugpumpe erzeugten und an dem Saugkanal (8, 9, 10, 15) anliegenden Saugdruck beeinflussen läßt;
gekennzeichnet durch
- eine Kupplungseinrichtung (7), die für eine axiale Verstellbarkeit der, in dem Führungsrohr (5) aufgenommenen Sonde (2) des Lithotripters (1) relativ zu dem Führungsrohr (5) ausgebildet ist, wobei über diese Kupplungseinrichtung ein Anschlußstück (6) des Führungsrohres (5) an einem Handstück (3) des Lithotripters lösbar zu befestigen ist und mit einer Durchgangsbohrung (9) des Anschlußstückes die mit der ersten Anschlußbohrung (10) verbundene Teillänge des Saugkanals (8, 9, 10, 15) ausgebildet wird.

2. Einrichtung nach Anspruch 1, dadurch gekennzeichnet, daß das Absperrorgan (11) als ein Regelorgan ausgebildet ist, mit dem bei angeschlossener Saugpumpe der an dem Saugkanal (8, 9, 10, 15) anliegende Saugdruck wahlweise geregelt oder abgesperrt werden kann.

3. Einrichtung nach Anspruch 2, dadurch gekennzeichnet, daß das Absperrorgan (11) als Trompetenventil ausgebildet ist.

4. Einrichtung nach einem der Ansprüche 1 bis 3, dadurch gekennzeichnet, daß das Anschlußstück (6) des Führungsrohres (5) einen koaxialen hohlen Gewindeansatz (13) aufweist, der mit einem Verbindungsstück (14) der Kupplungseinrichtung (7) verschraubt ist, wobei das Verbindungsstück eine mit der Durchgangsbohrung (9) des Anschlußstückes axial fluchtende zweite Anschlußbohrung (15) aufweist, welche an einem Luer-Lock-Anschluß (12') eine alternative Anschlußmöglichkeit für den Schlauchanschluß der Saugpumpe ergibt, wenn der Lithotripter (1) von der Einrichtung entfernt und die erste Anschlußbohrung durch das Absperrorgan (11) abgesperrt ist.

5. Einrichtung nach einem der Ansprüche 1 bis 4, dadurch gekennzeichnet, daß das Verbindungsstück (14) der Kupplungseinrichtung (7) an einem mit einem Außengewinde (16) versehenen Ansatz mit einer Kupplungshülse (17, 22) verschraubt ist, die mit dem Lithotripter (1) kraftschlüssig verbunden ist.

6. Einrichtung nach Anspruch 5, dadurch gekennzeichnet, daß in die Kupplungshülse (17, 22) ein Dichtungsring (18) eingesetzt ist, der durch ein Zusammenwirken der Kupplungshülse mit dem Verbindungsstück (14) der Kupplungseinrichtung (7) fixiert wird und den Saugkanal (8, 9, 10, 15) des Führungsrohres (5) gegen die durch den Dichtungsring (18) hindurchgeführte Sonde (2) des Lithotripters (1) abdichtet.

7. Einrichtung nach einem der Ansprüche 1 bis 6, dadurch gekennzeichnet, daß die Kupplungshülse (17) der Kupplungseinrichtung (7) über ein Stellgewinde (20) mit einer an dem Lithotripter (1) drehbar angeordneten Gewindehülse (19) verbunden ist, um durch eine Drehung der Gewindehülse relativ zu der Kupplungshülse die Sonde (2) des Lithotripters (1) relativ zu dem Führungsrohr (5) axial zu verstellen.

8. Einrichtung nach einem der Ansprüche 5 oder 6, dadurch gekennzeichnet, daß die Kupplungshülse (22) der Kupplungseinrichtung (7) an dem Lithotripter (1) axial verschiebbar angeordnet ist und ein Außengewinde (24) aufweist, mit welchem eine Überwurf-Spannmutter (23) verschraubt ist, um die Kupplungshülse gegen den Lithotripter zu verspannen.

9. Intrakorporaler Lithotripter (1) mit einer Stoßwellen übertragenden Sonde (2), die für eine Steinzertrümmerung in einen Arbeitskanal eines Endoskops eingesetzt wird, wobei der Lithotripter mit einer Einrichtung zum Entfernen von Körpersteinen gemäß einem oder mehreren der Ansprüche 1 bis 8 vereinigt ist.

## Claims

1. Device for removal of calculi including nephroliths, ureteroliths or urinary calculi, using an intracorporeal lithotripter (1) having a probe (2) which transmits shock waves and which for a lithotripsy is inserted into the operating passage of an endoscope, comprising
- a guiding tube (5) having an external diameter and an internal diameter where the external diameter is adapted for a coaxial insertion of the guiding tube (5) into the operating passage of the endoscope and the internal diameter is adapted for coaxially receiving the probe (2) of the lithotripter (1);
- a suction passage (8, 9, 10, 15) which is formed as an annular passage (8) between the guiding tube (5) and the probe (2) of the lithotripter (1) when inserted into the guiding tube whereby with a partial length (9) of the suction passage a first connecting bore (10) is connected as extending transversely or at an inclination with respect to the axis of this partial length is connected and which connecting bore may be connected to a tube connexion of a suction pump; and
- a shut-off member (11) which is arranged at the first connecting bore (10) and which allows to influence the suction pressure produced by the suction pump when the suction pump is coupled and which suction pressure is applied to the suction passage (8, 9, 10, 15);
characterized by
- a coupling means (7) which is adapted for an axial adjustment of the probe (2) of the lithotripter (1) relative to the guiding tube (5) when received in the guiding tube (5) whereby via said coupling means a connecting element (6) of the guiding tube (5) may be detachably fixed to a handpiece (3) of the lithotripter and whereby with a through-bore (9) of the connecting element the partial length of the suction passage (8, 9, 10, 15) connected to the first connecting bore (10) is being formed.

2. Device according to claim 1, characterized in that the shut-off member (11) is formed as a control member allowing selectively to either control or shut-off the suction pressure applied to the suction passage (8, 9, 10, 15) when the suction pump is connected.

3. Device according to claim 2, characterized in that the shut-off member (11) is formed as a trumpet-type valve.

4. Device according to any of the claims 1 to 3, characterized in that the connecting element (6) of the guiding tube (5) has a coaxial hollow thread fitting (13) which is screwed to a connecting element (14) of the coupling means (7) whereby the connecting element has a second connecting bore (15) which is axially aligned with the through-bore (9) of the connecting element, said second connecting bore having a Luer lock connector (12') providing an alternative possibility for connecting the tube connexion of the suction pump when the lithotripter (1) is removed from the device and the first connecting bore is shut-off by the shut-off member (11).

5. Device according to any of the claims 1 to 4, characterized in that the connecting element (14) of the coupling means (7) is screwed on an extension having a male thread (16) with a coupling sleeve (17, 22) which is non-positively connected with the lithotripter (1).

6. Device according to claim 5, characterized in that a sealing ring (18) is inserted into the coupling sleeve (17, 22) which sealing ring is being fixed by the co-operation of the coupling sleeve with the connecting element (14) of the coupling means (7) and which seals the suction passage (8, 9, 10, 15) of the guiding tube (5) against the probe (2) of the lithotripter (1) when passed through the sealing ring (18).

7. Device according to any of the claims 1 to 6, characterized in that the coupling sleeve (17) of the coupling means (7) is connected through an adjusting thread (20) to a threaded sleeve (19) rotatably arranged on the lithotripter (1) for axially adjusting the probe (2) of the lithotripter (1) relative to the guiding tube (5) by a rotation of the threaded sleeve.

8. Device according to any of the claims 5 or 6, characterized in that the coupling sleeve (22) of the coupling means (7) is arranged as axially displaceable in the lithotripter (1) and has a male thread (24) with which a union clamping nut (23) is screwed for clamping the coupling sleeve against the lithotripter.

9. Intracorporeal lithotripter (1) comprising a probe (2) which transmits shock waves for a lithotripsy and which is inserted into an operating channel of an endoscope where the lithotripter is united with a device for removing calculi in accordance with one or several of the claims 1 to 8.

## Revendications

1. Dispositif à évacuer des calculs comme des calculs rénaux, urétraux ou urinaires, en utilisant un lithotriteur (1) intracorporel à une sonde (2) qui transmet des ondes de choc et qui est insérée pour le broyage des calculs dans le canal opératoire d'un endoscope, comprenant:
- un tube de guidage (5) à un diamètre extérieur et un diamètre intérieur, dont le diamètre extérieur est adapté pour une introduction coaxiale dudit tube de guidage (5) dans ledit canal opératoire de l'endoscope et le diamètre intérieur pour un logement coaxial de ladite sonde (2) du lithotriteur (1);
- un canal d'aspiration (8, 9, 10, 15) conçu sous forme d'un canal de ceinture (8) entre ledit tube de guidage (5) et ladite sonde (2) du lithotriteur (1), qui est insérée dans ledit tube de guidage, à raccord d'un tronçon (9) dudit canal d'aspiration à un premier alésage de raccord (10) orienté au travers de ou incliné à l'axe de ce tronçon, auquel alésage de raccord on peut raccord un raccord de tuyau d'une pompe aspirante; et
- un organe d'arrêt (11) disposé audit premier alésage de raccord (10), qui, à pompe aspirante raccordée, permet le réglage d'une pression d'aspiration qui est engendrée par ladite pompe aspirante et alimentée audit canal d'aspiration (8, 9, 10, 15);
caractérisé par
- un moyen accoupleur (7) qui est conçu à permettre l'ajustage axial de ladite sonde (2) du lithotriteur (1), qui est insérée dans ledit tube de guidage (5), relativement audit tube de guidage (5), à attache mobile d'une pièce de jonction (6) dudit tube de guidage (5) via ce moyen accoupleur à une manette (3) du lithotriteur, et à formation d'un tronçon dudit canal d'aspiration (8, 9, 10, 15) moyennant un alésage de passage (9), lequel tronçon est raccordé audit premier alésage de raccord (10).

2. Dispositif selon la revendication 1, caractérisé en ce que ledit organe d'arrêt (11) est configuré sous forme d'un moyen de réglage approprié à permettre, a volonté, le réglage ou la fermeture de la pression d'aspiration appliquée, à pompe aspirante raccordée, audit canal d'aspiration (8, 9, 10, 15).

3. Dispositif selon la revendication 2, caractérisé en ce que ledit organe d'arrêt (11) a la configuration d'une soupape épanouie.

4. Dispositif selon une quelconque des revendications 1 à 3, caractérisé en ce que ladite pièce de jonction (6) dudit tube de guidage (5) présente une embase filetée creuse coaxiale (13) vissée à un connecteur (14) du moyen coupler (7), ledit connecteur comprenant un deuxième alésage de raccord (15) en alignement axiale audit alésage de passage (9) du connecteur, lequel deuxième alésage de raccord forant une deuxième alternative de raccord pour le raccord du tuyau de la pompe aspirante, à un raccord Luer-Lock (12') quand le lithotriteur (1) est démonté du dispositif et le premier alésage de raccord est fermé par ledit organe d'arrêt.

5. Dispositif selon une quelconque des revendications 1 à 4, caractérisé en ce que ledit connecteur (14) dudit moyen accoupleur (7) est vissé, à une embase à filet extérieur (16), à un manchon accoupleur (17, 22) qui est raccordé audit lithotriteur (1) par l'influence de force.

6. Dispositif selon la revendication 5, caractérisé en ce qu'un anneau de garniture (18) est inséré dans ledit manchon-accoupleur (17, 22), qui est fixé par l'interaction entre le manchon-accoupleur et le connecteur (14) du moyen accoupleur (7) et qui rend ledit canal d'aspiration (8, 9, 10, 15) dudit tube de guidage (5) étanche relativement à la sonde (2) du lithotriteur (1), qui est passée à travers ledit anneau de garniture (18).

7. Dispositif selon une quelconque des revendications 1 à 6, caractérisé en ce que ledit manchon-accoupleur (17) du moyen accoupleur (7) est raccordé, via un filet de régulation (20), à une douille taraudée (19) disposée audit lithotriteur de façon rotatoire pour l'ajustage axial de ladite sonde (2) du lithotriteur (1) relativement audit tube de guidage (5) par rotation de ladite douille taraudée relativement audit manchon-accoupleur.

8. Dispositif selon une quelconque des revendications 5 ou 6, caractérisé en ce que ledit manchon accoupleur (22) dudit moyen accoupleur (7) est disposé audit lithotriteur (1) pour un déplacement axial et comprend un filet extérieur (24) auquel est vissé un écrou-raccord tendeur (23) afin de tendre ledit manchon accoupleur audit lithotriteur.

9. Lithotriteur intracorporel (1) à une sonde (2) qui transmet des ondes de choc, qui est insérée dans un canal opératoire d'un endoscope pour le broyage des calculs, ce lithotriteur étant combiné avec un dispositif à évacuer des calculs selon une ou plusieurs des revendications 1 à 8.
